(19) Europäisches Patentamt
European Patent Office
Office européen des brevets

(11) **EP 4 631 365 A1**

(12) **EUROPEAN PATENT APPLICATION**
published in accordance with Art. 153(4) EPC

(43) Date of publication:
**15.10.2025  Bulletin 2025/42**

(21) Application number: 23912981.0

(22) Date of filing: 28.12.2023

(51) International Patent Classification (IPC):
**A23P 30/00** (2016.01)    **A23L 3/3544** (2006.01)
**A23L 27/30** (2016.01)

(52) Cooperative Patent Classification (CPC):
**A23B 2/771; A23L 27/30; A23P 30/00**

(86) International application number:
**PCT/KR2023/021852**

(87) International publication number:
**WO 2024/144307 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  28.12.2022  KR 20220187654

(71) Applicant: **CJ CheilJedang Corporation
Seoul 04560 (KR)**

(72) Inventors:
• **PARK, Chun Il**
  **Seoul 04560 (KR)**
• **CHOI, Jung Hwa**
  **Seoul 04560 (KR)**
• **JEONG, Daeyoung**
  **Seoul 04560 (KR)**
• **YU, Jaehun**
  **Seoul 04560 (KR)**

(74) Representative: **Meissner Bolte Partnerschaft
mbB
Patentanwälte Rechtsanwälte
Postfach 86 06 24
81633 München (DE)**

(54) **METHOD FOR PREPARING FERMENTATION PRODUCT**

(57)    The present disclosure relates to a method for producing a fermentation product, comprising: drying a process liquid containing a fermentation product to obtain the fermentation product, wherein the drying of the process liquid is performed by calculating the glass transition temperature of the process liquid based on the mass-based content of the fermentation product in the process liquid and the glass transition temperature of the fermentation product, and setting a drying process temperature of the process liquid based on the glass transition temperature of the process liquid. According to the present disclosure, errors during the drying process can be reduced, and fermentation products can be efficiently produced by setting the optimal drying temperature according to the type and content of the fermentation products to be produced.

[FIG. 1]

```
                    ┌─────────┐
                    │  Start  │
                    └────┬────┘
                         │
         ┌───────────────▼──────────────────────────┐
         │ Preparing process liquid containing       │~ S100
         │ fermentation product                      │
         └───────────────┬──────────────────────────┘
                         │
         ┌───────────────▼──────────────────────────┐
         │ Calculating process liquid drying         │~ S200
         │ conditions and obtaining fermentation     │
         │ product after drying process liquid       │
         └───────────────┬──────────────────────────┘
                         │
                    ┌────▼────┐
                    │   End   │
                    └─────────┘
```

**Description**

**[Technical Field]**

The present disclosure relates to a method for producing a fermentation product including a drying process.

**[Background Art]**

Fermentation products refer to useful substances produced using microorganisms or fungi, *etc.* Typical examples of fermentation products are amino acids or nucleic acids.

For amino acids and nucleic acids, existing crystalline powder-type and concentrated liquid-type products are commercially available. In the case of existing crystalline powder-type products, there is a limitation in product yield because it is difficult to completely recover the products from solvents, and products with low thermal stability may be easily deformed due to drying at high temperatures to remove the solvents on the product surface. In contrast, spray-dried products have high product yields because solvents, in which solutes are dissolved, are completely volatilized, and have an advantage in pulverization of substances with weak thermal stability because of their small particle size and short time of exposure to heat during drying.

Accordingly, research on commercialization of dried fermentation products is being carried out in various fields. However, in the case of fermentation products, especially amino acids, drying characteristics are affected by the content and purity of each substance and the type and mixing amount of excipients, and thus, product quality and product yields are affected depending on drying conditions; therefore, precise adjustment is necessary.

In the past, the drying process conditions were adjusted empirically, but as the types of fermentation products become more diverse and the types of added substances, such as excipients, also vary greatly, there are limitations in determining the process conditions empirically. In addition, it is not cost-effective when determining process conditions empirically and confirmation thereof through trial and error, until the process conditions are established.

**[Disclosure]**

**[Technical Problem]**

The present disclosure has been designed to implement the optimal drying process conditions for effective production of fermentation products prepared by a fermentation process.

**[Technical Solution]**

It is one object of the present disclosure to provide a method for effectively producing fermentation products prepared by a fermentation process.

It is another object of the present disclosure to provide a system for implementing the optimal drying process conditions for the production of fermentation products.

**[Advantageous Effects]**

According to the present disclosure, it is possible to produce high-quality fermentation products with high yield by finding the optimal spray drying conditions for each product through a spray drying temperature calculation equation based on the material information of each desired fermentation product. In particular, even with low thermal stability of fermentation products, the products can be stably produced with high yield through spray drying, in which the product characteristics are considered.

**[Brief Description of Drawings]**

FIG. 1 is a flowchart showing a method for producing a fermentation product according to the present disclosure.
FIG. 2 is a cross-sectional view showing a spray drying device according to one aspect of the present disclosure.
FIG. 3 is a flowchart showing a method for calculating the drying process temperature of process liquid according to one aspect of the present disclosure.
FIG. 4 is a graph showing the spray drying process yield according to the outlet temperature difference ($\Delta$T) of the spray drying process according to Experimental Example 1.

FIG. 5 is a graph analyzing the relationship of the difference (ΔT) between the outlet temperature difference of the spray drying process and the glass transition temperature of the process liquid calculated according to one aspect of the present disclosure, with the yield of the spray drying process.

**[Detailed Description of Preferred Embodiments]**

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

According to present disclosure, the glass transition temperature of the process liquid may be calculated based on the mass-based content of the fermentation product contained in the process liquid and the glass transition temperature of the fermentation product to be produced, and then the drying process temperature of the process liquid may be determined based on the calculated glass transition temperature of the process liquid. Accordingly, the optimal process conditions that can increase the process yield and stably powder the fermentation products may be implemented without multiple trials and errors.

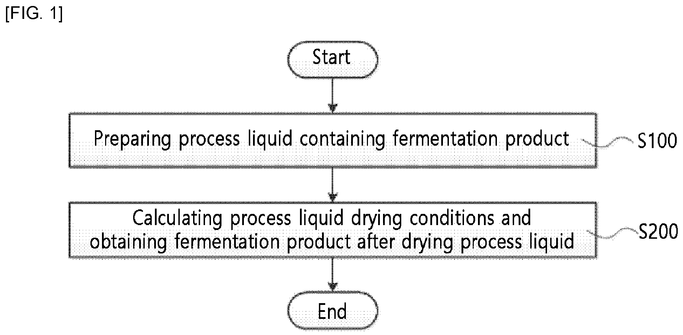

FIG. 1 is a flowchart showing a method for producing a fermentation product according to the present disclosure.

With reference to FIG. 1, the method for producing a fermentation product according to the present disclosure includes drying a process liquid containing a fermentation product to obtain the fermentation product. Hereinafter, each step will be described in detail.

With reference to FIG. 1, in order to produce a fermentation product, a step of preparing a process liquid containing the fermentation product **(S100)** is first performed.

The fermentation product contained in the process liquid prepared in the step of preparing the process liquid **(S100)** may be amino acids or nucleic acids. When the fermentation product contained in the process liquid is an amino acid, the amino acid may be at least one selected from the group consisting of glycine, alanine, serine, proline, valine, threonine, cysteine, isoleucine, leucine, asparagine, aspartic acid, glutamine, lysine, glutamic acid, methionine, histidine, phenylalanine, selenocysteine, arginine, tyrosine, and tryptophan. When the fermentation product is a nucleic acid, the nucleic acid may refer to a compound consisting of a base, sugar, and phosphate. Specifically, in the present disclosure, the nucleic acid may be any one or more selected from the group consisting of 5'-guanosine monophosphate (5'-GMP) and 5'-inosine monophosphate (5'-IMP). The fermentation products described above are exemplary, and the present disclosure can also be applied to the production of fermentation products other than the examples described above. This is because in the present disclosure, the drying process temperature is calculated based on the glass transition temperature, which is a unique characteristic of the fermentation product, and the content of the fermentation product.

In the step of preparing the process liquid **(S100),** a single type of fermentation product or a mixture of multiple types of fermentation products may be provided. If the glass transition temperature of each fermentation product provided in the process liquid and the content of the fermentation product in the process liquid are known, the glass transition temperature of the process liquid may be calculated, and thus, even when multiple types of fermentation products are mixed, the optimization of the process temperature may be performed according to the present disclosure.

The process liquid in the step of preparing the process liquid **(S100)** may mean a fermentation liquid containing the above-mentioned fermentation product. As used herein, the "fermentation liquid" may refer to a product resulting from enzymatic or metabolic decomposition of organic substances using a microorganism. For example, the fermentation liquid may include the culture itself obtained by culturing a microorganism in a culture medium, or a concentrate, dried product, or freeze-dried product of the culture obtained by removing the strain therefrom. In addition, in this case, the fermentation liquid may include the entire fermentation liquid containing the fermentation product, or may be one in which impurities have been removed from the fermentation liquid including the fermentation product.

**The** "microorganism for producing the fermentation product" used in the step of preparing process liquid **(S100)** or "microorganism for producing the fermentation product or desired product" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.,* and may be a microorganism including genetic modification to produce a desired protein or fermentation product.

The microorganism for producing the fermentation product of the present disclosure may be a microorganism naturally having a particular fermentation product producing ability, or a microorganism, in which the fermentation product producing ability has been imparted to a parent strain having no fermentation product producing ability, but is not limited thereto. Specifically, as used herein, the microorganism for producing the fermentation product or desired product, or the microorganism having the fermentation product or desired product producing ability may be a microorganism in which some genes in the biosynthesis pathway of the desired protein or desired product are enhanced or weakened, or some genes in the degradation pathway of the desired protein or desired product are enhanced or weakened. The "enhancement" or "increase" in the fermentation product producing ability of the microorganism of the present disclosure may mean that the particular fermentation product producing ability of the microorganism of the present disclosure is enhanced compared to that of a microorganism other than the microorganism of the present disclosure, a parent strain, or a non-modified microorganism. In one example, the microorganism of the present disclosure may have an enhanced producing ability by about 1% or more, 10% or more, 100% or more 200% or more 500% or more 1000% or more, 1100% or more, 1200% or more, 1300% or more, or about 1.01 times or more, 2 times or more, 5 times or more, 10 times or more, 11 times or more, 12 times or more, or 13 times or more compared to the particular fermentation product producing ability of other microorganisms, but is not limited thereto. As used herein, the term "about" refers to a range including all of $\pm 0.5$, $\pm 0.4$, $\pm 0.3$, $\pm 0.2$, $\pm 0.1$, *etc.,* and it includes all of the values equivalent to those which come immediately after the term "about" or those in a similar range, but is not limited thereto.

The above-mentioned microorganism used in the step of preparing the process liquid **(S100)** may be at least one selected from the group consisting of *Candida famata,* which is a yeast, *Eremothecium ashbyii* and *Ashbyagossypii,* which are ascomycetes, *Bacillus subtilis* and microorganisms belonging to the genus *Corynebacterium,* which are bacteria.

When the microorganism used in the step of preparing the process liquid **(S100)** is a microorganism belonging to the genus *Corynebacterium,* the microorganism may specifically be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium crenatum,* or *Corynebacterium flavescens,* more specifically *Corynebacterium glutamicum,* but is not limited thereto.

The step of preparing the process liquid **(S100)** may further include culturing "the microorganism for producing the fermentation product". The culturing of the microorganism may be performed in a suitable culture medium and culture conditions known in the art. Such a culturing process may be easily adjusted for use by those skilled in the art according to the strain to be selected. Specifically, the culturing may be a batch culture, a continuous culture, and/or a fed-batch culture, but is not limited thereto. As used herein, the term "medium" refers to a mixture of materials which contains nutrient materials required for culturing of the microorganism as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, while adjusting temperature, pH, *etc.*

The step of preparing the process liquid **(S100)** may further provide an excipient in addition to the above-described fermentation product in the fermentation liquid. The excipient may include, for example, preservatives, wetting agents, dispersing agents, suspending agents, buffers, stabilizers, or isotonic agents, *etc.,* but are not limited thereto. In addition, the excipient may be a non-naturally occurring substance or naturally occurring substance, but is not limited thereto.

The excipient mixed in the step of preparing the process liquid **(S100)** may be an excipient that is permitted to be added to food. For example, the excipient may be at least one selected from the group consisting of cross-linked sodium carboxymethyl cellulose, gum ghatti, persimmon color, licorice extract, formic acid, geranyl formate, citronellyl formate, isoamyl formate, masticatory substances, geraniol, microcrystalline cellulose, cinnamic acid, methyl cinnamate, ethyl cinnamate, cinnamaldehyde, cinnamyl alcohol, kaoliang color, benzoyl peroxide, hydrogen peroxide, peroxyacetic acid, ammonium persulfate, guar gum, disodium 5'-guanylate, citric acid, manganese citrate, trisodium citrate, sodium ferrous citrate, ferric citrate, ferric ammonium citrate, potassium citrate, calcium citrate, magnesium silicate, calcium silicate, silicone resin, diatomaceous earth, gluconic acid, sodium gluconate, copper gluconate, magnesium gluconate, manganese gluconate, zinc gluconate, ferrous gluconate, potassium gluconate, calcium gluconate, glutaminase, butyric acid, butyl butyrate, ethyl butyrate, isoamyl butyrate, neotame, nisin, nicotinic acid, nickel, nicotinamide, dextranase, dextran, sodium lauryl sulfate, lactase, lactoferrin concentrates, lactitol, lecithin, rosin, locust bean gum, rutin, linalool, mannitol, maltol, D-maltitol, sodium metasilicate, sodium metaphosphate, potassium metaphosphate, sodium metabisulfite, potassium metabisulfite, sodium methoxide, sulfur dioxide, myristic acid, microfibrillated cellulose, vanillin, kaolin, betaine, bentonite, powdered cellulose, sodium fluoride, biotin, vitamins, glacial acetic acid, DL-malic acid, sodium saccharin, saffron color, acid clay, sodium bisulfite, acidic sodium aluminum phosphate, disodium dihydrogen pyrophosphate, calcium dihydrogen pyrophosphate, magnesium oxide, zinc oxide, calcium oxide, methyl salicylate, iron sesquioxide, petroleum wax, sodium hydroxide, potassium hydroxide, calcium hydroxide, magnesium hydroxide, sucralose, shellac, steviol glycoside, stearic acid, sodium stearate, food colorings, benzoic acid, sodium benzoate, alginic acid and sodium alginate, inositol, silicon dioxide, chlorine dioxide, carbon dioxide, titanium dioxide, xanthan gum, lactic acid and sodium lactate, gelatin, gellan gum, aspergillus, carnauba wax, carrageenan, karaya gum, carotene, sodium carboxymethyl cellulose, calcium carboxymethyl cellulose, sodium carboxymethyl starch, casein and sodium caseinate, chitosan, chitin, tara gum, tamarind gum, taurine, tannic acid, palmitic acid, ethyl phenylacetate, isobutyl phenylacetate, pectin, pepsin, hydroxypropylmethyl cellulose, hydroxypropyl cellulose, hyaluronic acid, and enzyme extracts.

In the step of preparing the process liquid **(S100),** filtration and decolorization processes may be additionally performed on the fermentation liquid. Impurities may be additionally removed through filtration and decolorization. Filtration and decolorization processes may be performed by a conventional method, and may be omitted if necessary to simplify the process. In the step of preparing the process liquid **(S100),** a step of removing strains may be additionally performed. Removal of strains may be performed by various methods such as filtration, centrifugation, *etc.* Additionally, in the step of preparing the process liquid **(S100),** a desalting process may be performed. The desalting process may be performed to remove ionic impurities other than the fermentation product to be produced. The desalting process may be performed by various methods, such as ion exchange resins, continuous chromatography, *etc.* Further, in the step of preparing the process liquid **(S100),** a concentration process may be additionally performed. The concentration process may increase the concentration of fermentation products in the process liquid and enables to more easily obtain the fermentation products in the subsequent drying process. There are no limitations of performing the concentration process, and various methods such as rotary concentrator, *etc.,* may be used.

The order of filtration, decolorization, strain removal, and desalting described above in the step of preparing the process liquid **(S100)** may be varied as needed. The filtration, decolorization, and desalting may be performed after removal of strains, or the strains may be removed after filtration, decolorization, and desalting. Through the above-described filtration, decolorization, strain removal, and desalting, impurities other than fermentation products in the process liquid may be removed, and thus the glass transition temperature of the process liquid may be calculated more accurately in the next step.

Next, for the prepared process liquid, a step of calculating the drying conditions of the process liquid and drying the process liquid according to the calculated conditions to obtain a fermentation product is performed **(S200).**

Drying of the process liquid may be performed by various methods, but a spray drying method may be used to obtain the fermentation product in powder form.

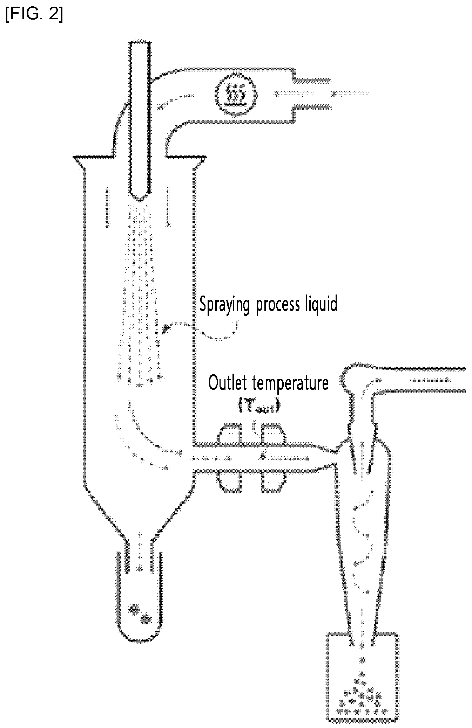

FIG. 2 is a cross-sectional view showing a spray drying device according to one aspect of the present disclosure. Spray drying is a method that can complete drying and granulation simultaneously, which directly dries solutions, emulsions and suspensions into powder or granular products, thereby eliminating evaporation, grinding and other processes. The fermentation products after spray drying may be dispersed into particles, or the fermentation products in the process liquid may be dried into a powder by removing most of the moisture

Spray drying is a method of instantly obtaining a dried product of liquid by spraying liquid in a heat stream at once, and may include centrifugal spraying using a rotating disk and pressurized spraying using a pressure nozzle, *etc.,* but is not limited thereto.

As can be confirmed in FIG. 2, during spray drying, the inlet temperature and outlet temperature of hot air may be set independently. In general, if spray drying is carried out above the glass transition temperature at which polymer materials vitrify (temperature of melting and fluidity), the material melts down in the spray dryer chamber and product discharge pipe, creating a sticky state. This not only can clog pipes, but also reduces the fluidity of the dried product, lowering the product recovery rate. To this end, in order to maintain an appropriate temperature for the spray drying process, the glass transition temperature of the product to be sprayed must be measured in advance, and the spray dryer outlet temperature condition must be set to a lower temperature than the glass transition temperature. Therefore, in the present disclosure, the drying conditions set to obtain a fermentation product by drying the process liquid may be the outlet temperature of the spray drying process.

In the case of fermentation products such as amino acids and organic acids, the molecular weight of the substance is less than 1000 mw, which is a small molecule, and it is difficult to measure or predict the accurate glass transition temperature because it is composed of a mixture of salts and other amino acids rather than a single substance. Therefore, in the present disclosure, the yield of spray drying was improved by deriving a correlation equation between the glass transition temperature and the molecular weight of the representative substances of each fermentation liquid and applying the equation as an alternative, and setting a temperature at which spray drying is possible.

When the amino acids and nucleic acids produced by fermentation are obtained as crystalline powders, it is difficult to completely recover the product from the solvent using a general drying process, thereby limiting the product yield. In addition, the fermentation products are dried at high temperatures to remove the solvent on the surface thereof, and products with low thermal stability may be easily deformed during this process. In contrast, spray-dried products have high product yields because solvents, in solutes are dissolved, are completely volatilized, and have an advantage in pulverization of substances with weak thermal stability because of their small particle size and short time to exposed to heat during drying. However, for some fermentation products such as fermented amino acids, the spray drying characteristics may vary depending on the content and purity of each substance, and the type and mixing amount of excipients. Therefore, the product quality and product yields are affected by the drying conditions, and in order to secure the product quality and yield, precise adjustment of drying conditions is necessary. According to the present disclosure, it is possible to produce high-quality fermentation products with high yield by finding the optimal spray drying conditions for each product through a spray drying temperature calculation equation based on the material information of each desired fermentation product. In particular, even with low thermal stability of fermentation products, the products can be stably produced with high yield through spray drying, in which the product characteristics are considered.

In the above, the process for obtaining a fermentation product from the process liquid containing the fermentation product according to one aspect of the present disclosure was examined. Hereinafter, a method for calculating drying process conditions for a process liquid containing a specific fermentation product will be described.

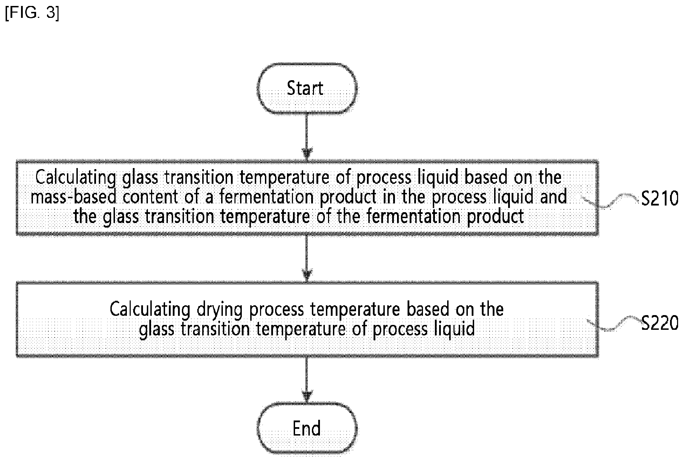

FIG. 3 is a flowchart showing a method for calculating the drying process temperature of a process liquid according to one aspect of the present disclosure.

With reference to FIG. 3, the drying process temperature of the process liquid is calculated by calculating the glass transition temperature of the process liquid based on the mass-based content of the fermentation product in the process liquid and the glass transition temperature of the fermentation product **(S210),** and setting the process temperature for drying the process liquid based on the glass transition temperature of the process liquid **(S220).**

First, in the step of calculating the glass transition temperature of the process liquid based on the mass-based content of the fermentation product in the process liquid and the glass transition temperature of the fermentation product **(S210),** the glass transition temperature of the process liquid is calculated according to Equation 1 below.

[Equation 1]

$$\frac{1}{T_{g,SD\ Feed}} = \frac{W_{fermentation\ liquid}}{T_{g,fermentation\ liquid}} + \frac{W_{excipient}}{T_{g\ excipient}}$$

(In Equation 1 above, $T_{g,SD\ Feed}$ is the glass transition temperature of the excipient-mixed process liquid, $W_{fermentation\ liquid}$ is the mass-based content of the fermentation product in the excipient-mixed process liquid, $T_{g,fermentation\ liquid}$ is the glass transition temperature of the process liquid, and $w_{excipient}$ is the mass-based content of the excipient in the excipient-mixed process liquid, and $T_{g,excipient}$ is the glass transition temperature of the excipient).

If an excipient is not mixed in the process liquid, the value of $w_{excipient}$ in Equation 1 above becomes 0. In addition, when a plurality of fermentation products are provided in the process liquid, multiple sets of $w_{fermentation\ liquid}$ and $T_{g,fermentation\ liquid}$ are provided in Equation 1.

In order to determine the mass-based content of the fermentation product contained in the process liquid in the above-mentioned Equation 1, a sample of the process liquid may be collected, and chromatography, *etc.,* may be performed on the sample.

Meanwhile, the glass transition temperature ($T_{g,fermentation\ liquid}$) of the process liquid may be calculated from the molecular weight of the fermentation product. The present inventor of the present disclosure derived the correlation between the molecular weight of fermentation products and the glass transition temperature using the molecular weight of amino acids and nucleic acids, and the actual measured glass transition temperature values known from the literature (Equation 2).

[Equation 2]

$$T_{g,fermentation\ liquid}(K) = 0.0996 * MW_{main\ fermentation\ product\ in\ process\ liquid} + 328.47$$

(In Equation 2 above, $MW_{main\ fermentation\ product\ in\ process\ liquid}$ is the molecular weight of the main fermentation product contained in the process liquid.)

Next, the process temperature for drying the process liquid s set based on the glass transition temperature of the process liquid **(S220).**

The process temperature of the drying process may be set 20°C to 25°C higher than the glass transition temperature of the process liquid. Additionally, in particular, the process temperature of the drying process may mean the outlet temperature of the spray drying process. By setting the drying process temperature higher than the glass transition temperature of the process liquid as the above-mentioned temperature, the yield of the drying process may be increased, and deformation of the fermentation product by high temperature may be prevented.

In the above, the method for calculating the drying process temperature of the process liquid according to one aspect of the present disclosure was examined. The method for calculating the drying process temperature above-described drying may be implemented in the form of a simulation system for a fermentation product production process. The simulation system for a fermentation product production process may be implemented using hardware components, software components, or a combination of the hardware components and software components in order to increase the quality and yield of fermentation products by calculating the optimal drying conditions according to the type and content of fermentation products and the type and content of excipients. For example, the hardware components may include microphones, amplifiers, band-pass filters, audio to digital convertors, and processing devices. A processing device may be implemented using one or more generalpurpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that a processing device may include multiple processing elements and multiple types of processing elements. For example, a processing device may include multiple processors or a processor and a controller. In addition, different processing configurations are possible, such a parallel processor.

In addition, the method for calculating the drying process temperature of the process liquid described above can be implemented in a software form. The software may include a computer program, a piece of code, an instruction, or some combination thereof, to independently or collectively instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums. The methods according to the above-described embodiments may be recorded in non-transitory computer-readable media including program instructions to implement various operations embodied by a computer. The media may also include, alone or in combination with the program instructions, data files, data structures, and the like. The program instructions recorded on the media may be those specially designed and constructed for the purposes of the embodiments, or they may be of the kind well known and available to those having skill in the computer software arts. Examples of non-transitory computer-readable media include magnetic media such as hard disks, floppy disks, and magnetic tape; optical media such as CD ROM disks and DVDs; magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter.

**[Mode for Carrying Out the Invention]**

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

In the above, the fermentation product production process and the simulation system for a fermentation product production process according to one aspect of the present disclosure were examined. Hereinafter, the beneficial effects mentioned in the present disclosure will be described through the experimental results of Examples and Comparative Examples.

**Experimental Example 1. Confirmation of Difference between Glass Transition Temperature Calculated Through Molecular Weight of Fermentation Products and Glass Transition Temperature Confirmed Through Actual Measurement**

As previously examined, according to the present disclosure, the glass transition temperature ($T_g$, fermentation liquid) of the process liquid can be calculated from the molecular weight of the fermentation product. The present inventor of the present disclosure derived the correlation between the molecular weight of fermentation products and the glass transition temperature using the molecular weight of amino acids and nucleic acids and the actual measured glass transition temperature values known from the literature (Equation 2).

[Equation 2]

$$T_{g,\text{fermentation liquid}}(\text{K}) = 0.0996 * MW_{\text{main fermentation product in process liquid}} + 328.47$$

(In Equation 2 above, $MW_{\text{main fermentation product in process liquid}}$ is the molecular weight of the main fermentation product contained in the process liquid.)

In Experimental Example 1, the error between the glass transition temperature calculated according to Equation 2 and the glass transition temperature of the fermentation product confirmed through actual measurements was determined, and it was confirmed whether the glass transition temperature calculated according to Equation 2 was reliable.

With reference to Table 1, the difference between the actual and calculated values of $T_{g,\text{fermentation liquid}}$ is expressed as error, and when the correlation coefficient $R_2$, which is used to determine how accurate the model is, was calculated, it was found to be 0.9831. This is a value that satisfies the generally accepted criteria for determining the effectiveness of correlation coefficients (around 0.95 for bio, 0.7 for engineering, and 0.3 for social sciences), meaning that the glass transition temperature value calculated according to Equation 2 is a reliable value.

[Table 1]

| Main Materials in Process Liquid | Molecular Weight | $T_g$ Calculated Value | | $T_g$ Actual Value | Error | | | |
|---|---|---|---|---|---|---|---|---|
| | MW | K | °C | °C | (Calculation Equation - Actual Value) / Actual Value) % | Average | Standard Deviation | $R^2$ |
| Glycine | 75.07 | 335.95 | 62.80 | 60.85 | -3.21 | 0.07 | 1.39 | 0.9831 |
| Alanine | 89.09 | 337.34 | 64.19 | 64.08 | -0.17 | | | |
| Serine | 105.09 | 338.94 | 65.79 | 64.13 | -2.59 | | | |
| Threonine | 119.10 | 340.33 | 67.18 | 66.95 | -0.34 | | | |
| Cysteine | 121.20 | 340.54 | 67.39 | 68.68 | 1.87 | | | |
| Valine | 117.10 | 340.13 | 66.98 | 67.96 | 1.44 | | | |
| Leucine | 131.20 | 341.54 | 68.39 | 66.87 | -2.27 | | | |
| Isoleucine | 131.20 | 341.54 | 68.39 | 68.17 | -0.31 | | | |
| Methionine | 149.20 | 343.33 | 70.18 | 72.14 | 2.72 | | | |
| Proline | 115.10 | 339.93 | 66.78 | 66.84 | 0.09 | | | |
| Phenylalanine | 165.20 | 344.92 | 71.77 | 72.26 | 0.68 | | | |
| Tyrosine | 181.20 | 346.52 | 73.37 | 73.37 | 0.00 | | | |
| Tryptophan | 204.20 | 348.81 | 75.66 | 75.67 | 0.02 | | | |
| Aspartic acid | 133.10 | 341.73 | 68.58 | 68.58 | 0.00 | | | |
| Glutamic acid | 147.10 | 343.12 | 69.97 | 70.28 | 0.44 | | | |

(continued)

| Main Materials in Process Liquid | Molecular Weight | $T_g$ Calculated Value | | $T_g$ Actual Value | Error | | | |
|---|---|---|---|---|---|---|---|---|
| | MW | K | °C | °C | (Calculation Equation - Actual Value) / Actual Value) % | Average | Standard Deviation | $R^2$ |
| Asparagine | 132.10 | 341.63 | 68.48 | 68.22 | -0.38 | | | |
| Glutamine | 146.10 | 343.02 | 69.87 | 71.09 | 1.72 | | | |
| Histidine | 156.20 | 344.03 | 70.88 | 71.35 | 0.67 | | | |
| Lysine | 146.20 | 343.03 | 69.88 | 69.88 | 0.00 | | | |
| Arginine | 174.20 | 345.82 | 72.67 | 73.07 | 0.55 | | | |
| IMP | 348.21 | 363.15 | 90.00 | 91.32 | 1.44 | | | |
| GMP | 363.22 | 364.65 | 91.50 | 90.74 | -0.84 | | | |

## Experimental Example 2. Calculation of Glass Transition Temperature of Process Liquid Containing Glutamic Acid and Design of Drying Process Based on Calculated Glass Transition Temperature

Amino acid fermentation liquid with a concentration of glutamic acid of 5 wt% and a solid content of 10 wt% in the liquid phase was subjected to a membrane separation process to remove bacterial cells, and concentrated using a rotary concentrator to reach a concentration range of 15 wt% to 30 wt%. The resulting concentrate was subjected to through decolorization and filtration processes and mixed with an excipient (maltodextrin (DE20) $T_{g, excipient}$: 141°C) to prepare a spray drying process liquid having the glutamic acid content of 15 wt%, the excipient content of 16.7 wt%, and the solid content of 40 wt%.

The spray drying outlet temperature of the process liquid was calculated using Equations 1 and 2 confirmed above. Each term used in the equations is as follows: $W_{fermentation\ liquid}$: 0.833, $w_{excipient}$: 0.167, $MW_{main\ fermentation\ product\ in\ process\ liquid}$: 147.10, and $T_{g,\ fermentation\ liquid}$: 414.15 K. Using the above-mentioned values, the spray drying outlet temperature was calculated to obtain a value of 80.07°C, and when spray drying was performed under these conditions, the product recovery rate was around 80%.

## Experimental Example 3. Analysis of Process Yield According to Difference between Calculated Glass Transition Temperature and Spray Drying Process Outlet Temperature

In Experimental Example 3, the glass transition temperature was calculated for the process liquid of a specific composition, and the change in the process yield according to the temperature difference was confirmed when the difference between the calculated glass transition temperature and the spray drying process outlet temperature was varied.

In Experimental Example 3, the process yield was calculated as the mass of the product obtained relative to the amount of solids in the spray drying process liquid.

[Spray drying process yield (%) = Amount of spray dried product obtained (wt) / (Total amount of spray drying process liquid (wt) * Solid content (wt%)) * 100%]

[Table 2]

| Formulation | Preparation Example 1 |
|---|---|
| Type of Excipient | Potato Dextrin Glucidex |
| Glass Transition Temperature of Process Liquid ($T_g$) (°C) | 105.1 |

(continued)

| Formulation | Preparation Example 1 | | | | | | | | |
|---|---|---|---|---|---|---|---|---|---|
| Type of Excipient | Potato Dextrin Glucidex | | | | | | | | |
| Spray Drying Process Outlet Temperature ($T_{out}$) (°C) | 75 | 77 | 80 | 83 | 85 | 90 | 95 | 100 | 105 |
| $\Delta T$ (Tout - Tg) (°C) | 30.1 | 28.1 | 25.1 | 22.1 | 20.1 | 15.1 | 10.1 | 5.1 | 0.1 |
| Average Yield of Spray Drying Process (%) | 69.0 % | 76.6 % | 81.05 % | 79.54 % | 78.0 % | 69.9 % | 69.4 % | 50.8 % | 36.3 % |

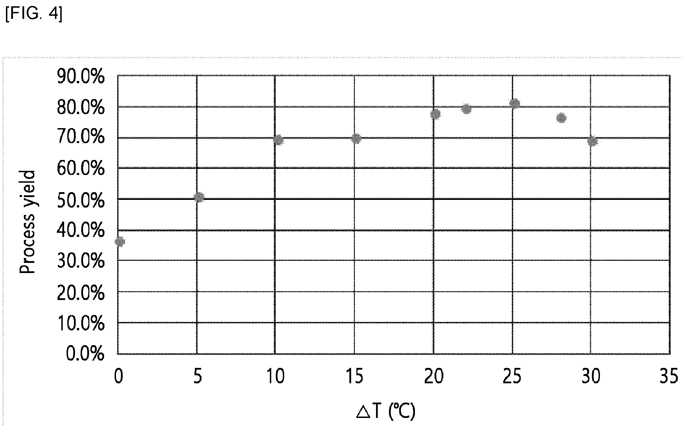

FIG. 4 is a graph showing the spray drying process yield according to the outlet temperature difference ($\Delta T$) of the spray drying process according to Experimental Example 3.

With reference to FIG. 4, when the outlet temperature difference ($\Delta T$) of the spray drying process was varied for the process liquid of the same composition, it can be confirmed that the process yield was high when the temperature difference was about 20°C to about 25°C.

**Experimental Example 4. Analysis of Process Yield According to Difference Between Glass Transition Temperature Calculated for Process Liquids with Different Types and Contents of Excipients and Spray Drying Process Outlet Temperature**

In Experimental Example 4, the effect of the difference between the glass transition temperature of the process liquids containing the fermentation products calculated by the method described above and the outlet temperature of the spray drying process on the spray drying process yield was analyzed.

Table 3 shows the spray drying process yield calculated according to the calculated glass transition temperature ($T_g$) according to the type of excipients contained in the process liquids containing glutamic acid as a fermentation product, and the difference ($\Delta T$) between the calculated glass transition temperature and the outlet temperature of the spray drying process. The spray drying process yield was calculated in the same manner as in Experimental Example 3.

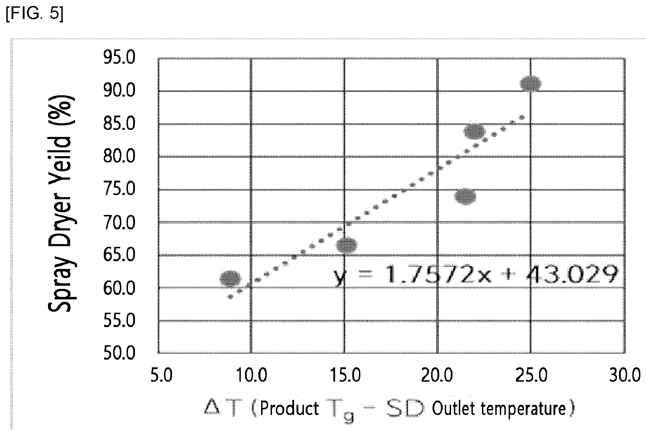

FIG. 5 is a graph analyzing the relationship of the difference ($\Delta T$) between the outlet temperature difference of the spray drying process and the glass transition temperature of the process liquid calculated according to one aspect of the present disclosure, with the yield of the spray drying process.

Table 3 shows the results of a spray drying experiment using the same glutamic acid fermentation liquid with different types and amounts of excipients.

[Table 3]

| Formulation | Preparation Example 1 | Preparation Example 2 | Preparation Example 3 | Preparation Example 4 | Preparation Example 5 |
|---|---|---|---|---|---|
| Type of Excipient | Glucidex | Glucidex | Chicory Fiber | Chicory Fiber | Chicory Fiber |
| Glutamic acid content (mass%) | 25 | 20 | 22.1 | 27 | 22 |
| Excipient content (mass%) | 45% | 56% | 66% | 41% | 67% |
| Spray Drying Process Outlet Temperature ($T_{out}$) (°C) | 90 | 90 | 80 | 80 | 80 |
| Glass Transition Temperature of Process Liquid ($T_g$) (°C) | 105.1 | 114.9 | 101.4 | 88.9 | 102.0 |
| $\Delta T$ (Tout - Tg) (°C) | 15.1 | 24.9 | 21.4 | 8.9 | 22.0 |
| Average Yield of Spray Drying Process (%) | 66.5 | 91.2 | 74.1 | 61.5 | 84.0 |

As can be confirmed in Table 3 and FIG. 5, regardless of the type and content of excipients, it was confirmed that the average yield of the spray drying process was high when $\Delta T$ was between 20°C and 25°C. In contrast, when $\Delta T$ was less than 20°C, it was confirmed that the average yield of the spray drying process decreased significantly. Therefore, it was confirmed that the spray drying process yield can be greatly improved by calculating the glass transition temperature of the process liquid and setting the spray drying process outlet temperature based thereon.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

## Claims

1. A method for producing a fermentation product, comprising: drying a process liquid containing a fermentation product to obtain the fermentation product,
   wherein the drying of the process liquid is performed by calculating the glass transition temperature of the process liquid based on the mass-based content of the fermentation product in the process liquid and the glass transition temperature of the fermentation product, and setting a drying process temperature of the process liquid based on the glass transition temperature of the process liquid.

2. The method of claim 1, wherein the drying of the process liquid is carried out by spray drying, and the drying process temperature of the process liquid is the outlet temperature of the spray drying process.

3. The method of claim 1, wherein the fermentation product comprises at least one selected from the group consisting of amino acids and nucleic acids.

4. The method of claim 1, wherein the process liquid further comprises an excipient, and the mass-based content of the excipient in the excipient-mixed process liquid and the glass transition temperature of the excipient are further considered in order to calculate the glass transition temperature of the excipient-mixed process liquid containing the excipient.

5. The method of claim 4, wherein the glass transition temperature of the process liquid is calculated using Equation 1 below

[Equation 1]

$$\frac{1}{T_{g,SD\ Feed}} = \frac{W_{fermentation\ liquid}}{T_{g,fermentation\ liquid}} + \frac{W_{excipient}}{T_{g\ excipient}}$$

(In Equation 1 above, $T_{g,\ SD\ Feed}$ is the glass transition temperature of the excipient-mixed process liquid, $W_{fermentation\ liquid}$ is the mass-based content of the fermentation product in the excipient-mixed process liquid, $T_{g,\ fermentation\ liquid}$ is the glass transition temperature of the process liquid, and $w_{excipient}$ is the mass-based content of the excipient in the excipient-mixed process liquid, and $T_{g,\ excipient}$ is the glass transition temperature of the excipient).

6. The method of claim 5, wherein the glass transition temperature of the process liquid ($T_{g,\ fermentation\ liquid}$) is calculated using Equation 2 below

[Equation 2]

$$T_{g,fermentation\ liquid}(K) = 0.0996 * MW_{main\ fermentation\ product\ in\ process\ liquid} + 328.47$$

(In Equation 2 above, $MW_{main\ fermentation\ product\ in\ process\ liquid}$ is the molecular weight of the main fermentation product contained in the process liquid).

7. The method of claim 1, wherein the process temperature of the drying process is set 20°C to 25°C higher than the glass transition temperature of the process liquid.

8. The method of claim 1, wherein the method further comprises removing bacteria from the process liquid and concentrating the process liquid before drying the process liquid.

9. A simulation system for a production process of a fermentation product, which calculates the glass transition temperature of a process liquid based on the mass-based content of a fermentation product in the process liquid and the glass transition temperature of the fermentation product, and determines a drying process temperature of the process liquid based on the glass transition temperature of the process liquid, in order to obtain the fermentation product by drying the process liquid containing the fermentation product.

10. A recording medium, in which software is recorded that calculates the glass transition temperature of a process liquid based on the mass-based content of a fermentation product in the process liquid and the glass transition temperature of the fermentation product, and determines a drying process temperature of the process liquid based on the glass transition temperature of the process liquid, in order to obtain the fermentation product by drying the process liquid containing the fermentation product.

[FIG. 1]

```
                    ┌─────────────┐
                    │    Start    │
                    └─────────────┘
                           │
                           ▼
┌──────────────────────────────────────────────────────┐
│  Preparing process liquid containing fermentation product │  ⌇ S100
└──────────────────────────────────────────────────────┘
                           │
                           ▼
┌──────────────────────────────────────────────────────┐
│        Calculating process liquid drying conditions and        │  ⌇ S200
│   obtaining fermentation product after drying process liquid   │
└──────────────────────────────────────────────────────┘
                           │
                           ▼
                    ┌─────────────┐
                    │     End     │
                    └─────────────┘
```

[FIG. 2]

Spraying process liquid

Outlet temperature
$(T_{out})$

[FIG. 3]

```
                    ┌──────────┐
                    │  Start   │
                    └────┬─────┘
                         │
                         ▼
┌──────────────────────────────────────────────────────────────┐
│ Calculating glass transition temperature of process liquid     │
│ based on the mass-based content of a fermentation product in    │──── S210
│ the process liquid and the glass transition temperature of the  │
│ fermentation product                                            │
└───────────────────────────┬────────────────────────────────────┘
                            │
                            ▼
┌──────────────────────────────────────────────────────────────┐
│ Calculating drying process temperature based on the            │──── S220
│ glass transition temperature of process liquid                 │
└───────────────────────────┬────────────────────────────────────┘
                            │
                            ▼
                    ┌──────────┐
                    │   End    │
                    └──────────┘
```

[FIG. 4]

[FIG. 5]

**EP 4 631 365 A1**

## INTERNATIONAL SEARCH REPORT

| | International application No. |
|---|---|
| | **PCT/KR2023/021852** |

**A. CLASSIFICATION OF SUBJECT MATTER**

**A23P 30/00**(2016.01)i; **A23L 3/3544**(2006.01)i; **A23L 27/30**(2016.01)i

According to International Patent Classification (IPC) or to both national classification and IPC

**B. FIELDS SEARCHED**

Minimum documentation searched (classification system followed by classification symbols)

A23P 30/00(2016.01); A23G 1/00(2006.01); A23G 1/46(2006.01); A23L 11/00(2016.01); A23L 2/38(2006.01); A61J 3/02(2006.01); A61K 31/198(2006.01); A61K 9/14(2006.01); A61K 9/16(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 발효(fermentation), 건조 온도(drying temperature), 유리전이온도(glass transition temperature), 중량(weight)

**C. DOCUMENTS CONSIDERED TO BE RELEVANT**

| Category* | Citation of document, with indication, where appropriate, of the relevant passages | Relevant to claim No. |
|---|---|---|
| Y | US 2022-0279807 A1 (MARS, INCORPORATED) 08 September 2022 (2022-09-08)<br>See claim 4; paragraphs [0013]-[0045]; and figure 2. | 1-10 |
| Y | 김병철 등. PMMA/PVDF 혼합물의 물성 및 광학적 성질. 폴리머. 2002, vol. 26, no. 4, pp. 462-467<br>(KIM, Byoung Choul et al. Physical and Optical Properties of PMMA / PVDF Blends. Polymer.)<br>See page 464. | 1-10 |
| Y | KR 10-2021-0025574 A (CJ CHEILJEDANG CORPORATION) 09 March 2021 (2021-03-09)<br>See claim 1. | 3,6,8 |
| A | KR 10-2010-0112206 A (MEDIMMUNE, LLC) 18 October 2010 (2010-10-18)<br>See entire document. | 1-10 |
| A | KR 10-2022-0012058 A (CHAMJAYEON, CO., LTD.) 03 February 2022 (2022-02-03)<br>See entire document. | 1-10 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 April 2024** | **09 April 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/021852**

| Patent document cited in search report | | | Publication date (day/month/year) | Patent family member(s) | | | Publication date (day/month/year) |
|---|---|---|---|---|---|---|---|
| US | 2022-0279807 | A1 | 08 September 2022 | CN | 114206121 | A | 18 March 2022 |
| | | | | CN | 114206122 | A | 18 March 2022 |
| | | | | EP | 4007496 | A1 | 08 June 2022 |
| | | | | EP | 4007496 | A4 | 16 August 2023 |
| | | | | EP | 4007498 | A2 | 08 June 2022 |
| | | | | EP | 4007498 | A4 | 13 September 2023 |
| | | | | US | 2022-0279809 | A1 | 08 September 2022 |
| | | | | WO | 2021-026089 | A2 | 11 February 2021 |
| | | | | WO | 2021-026089 | A3 | 11 March 2021 |
| | | | | WO | 2021-026103 | A1 | 11 February 2021 |
| KR | 10-2021-0025574 | A | 09 March 2021 | CN | 112135607 | A | 25 December 2020 |
| | | | | CN | 112135607 | B | 04 August 2023 |
| | | | | EP | 3756654 | A1 | 30 December 2020 |
| | | | | EP | 3756654 | A4 | 14 April 2021 |
| | | | | KR | 10-2019-0111835 | A | 02 October 2019 |
| | | | | KR | 10-2022-0017450 | A | 11 February 2022 |
| | | | | KR | 10-2223797 | B1 | 12 March 2021 |
| | | | | US | 11370746 | B2 | 28 June 2022 |
| | | | | US | 2021-0094903 | A1 | 01 April 2021 |
| | | | | WO | 2019-182413 | A1 | 26 September 2019 |
| KR | 10-2010-0112206 | A | 18 October 2010 | CN | 101287449 | A | 15 October 2008 |
| | | | | CN | 101287449 | B | 03 November 2010 |
| | | | | EP | 1572915 | A2 | 14 September 2005 |
| | | | | EP | 1572915 | A4 | 05 January 2011 |
| | | | | JP | 2006-512102 | A | 13 April 2006 |
| | | | | KR | 10-2005-0011741 | A | 29 January 2005 |
| | | | | US | 2003-0215515 | A1 | 20 November 2003 |
| | | | | US | 2007-0259334 | A1 | 08 November 2007 |
| | | | | US | 2008-0131514 | A1 | 05 June 2008 |
| | | | | US | 2012-0009248 | A1 | 12 January 2012 |
| | | | | US | 7258873 | B2 | 21 August 2007 |
| | | | | US | 7700130 | B2 | 20 April 2010 |
| | | | | US | 8012507 | B2 | 06 September 2011 |
| | | | | US | 8273374 | B2 | 25 September 2012 |
| | | | | WO | 03-087335 | A2 | 23 October 2003 |
| | | | | WO | 2003-087335 | A3 | 03 January 2008 |
| KR | 10-2022-0012058 | A | 03 February 2022 | KR | 10-2614506 | B1 | 26 December 2023 |

Form PCT/ISA/210 (patent family annex) (July 2022)